# EUROPÄISCHE PATENTANMELDUNG

(11) **EP 3 799 062 A1**
(43) Veröffentlichungstag der Anmeldung: **31.03.2021**
(21) Anmeldenummer: 19199605.7
(22) Anmeldetag: 25.09.2019
(51) Int. Cl.: G16H 30/40, G16H 40/20, G16H 50/20

(54) **VERFAHREN ZUM BESTIMMEN EINES BEVORZUGTEN ZIELORTS FÜR EIN FAHRZEUG**

(71) Anmelder: Siemens Healthcare GmbH, 91052 Erlangen (DE)
(72) Erfinder: Lautenschläger, Stefan, 90762 Nürnberg (DE)

(57) **Zusammenfassung**

Die Erfindung betrifft ein Verfahren zum Bestimmen eines bevorzugten Zielorts für ein Fahrzeug (100), insbesondere einen Krankenwagen, aufweisend ein medizinisches Bildgebungsgerät (101), umfassend die Schritte,
- Bereitstellen (S1) eines Bilddatensatzes eines Patienten (103), welcher mittels des medizinischen Bildgebungsgeräts (101) erzeugt wurde, mittels einer ersten Schnittstelle (105),
- Bewerten (S2) des bereitgestellten Bilddatensatzes mittels einer Recheneinheit (107),
- Erstes Bestimmen (S3) einer Position (4) des Fahrzeugs (100) mittels der Recheneinheit (107),
- Zweites Bestimmen (S4) einer Anzahl an optionalen Zielorten (1,2,3) für eine Behandlung des Patienten zumindest basierend auf der bestimmten Position (4) des Fahrzeugs (100) mittels der Recheneinheit (107),
- Drittes Bestimmen (S5) des bevorzugten Zielorts aus der Anzahl an optionalen Zielorten (1,2,3) zumindest basierend auf dem bewerteten Bilddatensatz und der bestimmten Position (4) des Fahrzeugs (100) mittels der Recheneinheit (107),
- Ausgeben (S6) des ausgewählten, bevorzugten Zielorts mittels einer zweiten Schnittstelle (109).

## Beschreibung

Die Erfindung betrifft ein Verfahren zum Bestimmen eines bevorzugten Zielorts für ein Fahrzeug aufweisend ein medizinisches Bildgebungsgerät, sowie ein Fahrzeug, ein Computerprogrammprodukt, ein Computerprogramm und ein computerlesbares Speichermedium.

Für die Behandlung von einem Traumapatienten oder einen Schlaganfallpatienten wird eine Diagnose typischerweise durch einen Arzt und mit Hilfe von medizinischen Bilddatensätzen durchgeführt. Für eine vollständige Analyse der medizinischen Situation eines Patienten, werden häufig dreidimensionale Volumenbilddaten mittels eines Computertomographie-Geräts (CT-Gerät) breitgestellt. Das bedeutet, der Bildgebungsprozess besteht auf einer Anzahl an Projektionsmessdaten, welche aus unterschiedlichen Winkelbereichen aufgenommen werden und einer folgenden mathematischen Rücktransformation um die dreidimensionalen Volumenbilddaten basierend auf den Projektionsmessdaten zu rekonstruieren (z.B. mittels eines Rekonstruktionsalgorithmus der gefilterten Rückprojektion). Ausgehend von diesen dreidimensionalen Volumenbilddaten können auch zweidimensionale Schichtbilder, welche jeweils ein Schnittbild durch das abgebildete Volumen darstellen, erzeugt werden.

Im Falle eines schweren Traumas oder Schlaganfalls ist Zeit ein wichtiger Faktor. Je früher die richtige Behandlung beginnt, desto besser sind die Aussichten auf einen Behandlungserfolg. Momentane Entwicklungen ermöglichen für eine möglichst zeitnahe Untersuchung und Diagnose flexiblere Einsätze von CT-Geräten, beispielsweise in Krankenwägen (sogenannte "mobile stroke units", mobile Schlaganfalleinheiten) oder sogar in Helikoptern. Dabei ist jedoch typischerweise kein für die diagnostische Auswertung der Bilddatensätze ausgebildeter Arzt, beispielsweise ein Neuroradiologe, im Krankenwagen vorgesehen, so dass die aufgenommenen Bilddatensätze für eine Diagnose beispielsweise an ein Krankenhaus übertragen werden müssen.

Die Datenübertragung, meist über Mobilfunkverbindung, stellt meist jedoch einen zeitintensiven Faktor dar, welcher eine zeitnahe Beurteilung durch einen Arzt verzögert. Je nach Ergebnis der Beurteilung können jedoch die weiteren, notwendigen Schritte für die Behandlung eines Patienten unterschiedlich ausfallen. Ebenso sind je nach notwendiger Behandlung unterschiedliche Behandlungsorte, beispielsweise je nach ihrer Ausstattung und Verfügbarkeit, für den Behandlungserfolg oder auch hinsichtlich einer Kosteneffizienz zu bevorzugen.

Beispielsweise können Krankenhäuser bzw. Schlaganfallzentren (sogenannte Stroke Units bzw. Stroke Center) in unterschiedliche Ausstattungs- bzw. Behandlungslevel hinsichtlich der Behandlungsmöglichkeiten und vorliegenden Strukturen für die Behandlung von Schlaganfallpatienten eingestuft werden. So können insbesondere in den USA primäre Schlaganfallzentren (Primary Stroke Center, PSC) von umfangreicher ausgestatteten Schlaganfallzentren (Comprehensive Stroke Center, CSC) bzw. Schlaganfallzentren, welche für eine Thrombektomie ausgestattet sind (Thrombectomy Capable Stroke Center, TCSC) unterschieden werden. Ähnliche Abstufungssysteme gibt es auch in anderen Ländern und Regionen. Ein Schlaganfallpatient, welcher beispielsweise einen Gefäßverschluss einer großen Gehirnarterie (Large Vessel Occlusion, LVO), aufweist, benötigt einen anderen Ausstattungslevel, insbesondere ein CSC/TCSC-Level, als ein Schlaganfallpatient, bei welchem dies nicht der Fall ist und ein PSC-Level für die weiterführende Behandlung ausreicht und dabei wesentlich kosteneffizienter ist, oder bei dem sich sogar der Verdacht auf einen Schlaganfall nicht bestätigt.

Aufgabe der Erfindung ist es daher ein verbessertes Verfahren zum Bestimmen eines bevorzugten Zielorts für ein Fahrzeug, insbesondere einen Krankenwagen, aufweisend ein medizinisches Bildgebungsgerät bereitzustellen, um eine möglichst optimale Behandlung eines Patienten zu ermöglichen.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Nachstehend wird die erfindungsgemäße Lösung der Aufgabe sowohl in Bezug auf das beanspruchte Verfahren als auch in Bezug auf eine erfindungsgemäße Vorrichtung in Form des beanspruchten Fahrzeugs beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung, d.h. auf das beanspruchte Fahrzeug, gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein Verfahren zum Bestimmen eines bevorzugten Zielorts für ein Fahrzeug, insbesondere einen Krankenwagen, aufweisend ein medizinisches Bildgebungsgerät, umfassend das Bereitstellen eines mittels des medizinischen Bildgebungsgeräts erzeugten Bilddatensatzes eines Patienten mittels einer ersten Schnittstelle und das Bewerten des bereitgestellten Bilddatensatzes mittels einer Recheneinheit. Weiterhin umfasst das erfindungsgemäße Verfahren ein erstes Bestimmen einer Position des Fahrzeugs mittels der Recheneinheit, ein zweites Bestimmen einer Anzahl an optionalen Zielorten für eine Behandlung des Patienten zumindest basierend auf der bestimmten Position des Fahrzeugs mittels der Recheneinheit, und ein drittes Bestimmen des bevorzugten Zielorts aus der Anzahl an optionalen Zielorten zumindest basierend auf dem bewerteten Bilddatensatz und der bestimmten Position des Fahrzeugs mittels der Recheneinheit. Daran schließt sich ein Schritt des Ausgebens des ausgewählten, bevorzugten Zielorts mittels einer zweiten Schnittstelle an.

Der Erfindung liegt die Überlegung zugrunde, dass im Rahmen eines Einsatzes eines Fahrzeugs, insbesondere eines Krankenwagens, aufweisend ein medizinisches Bildgebungsgerät zumindest basierend auf einer ersten, vorzugsweise automatisch mittels der Recheneinheit durchgeführten Bewertung des bereitgestellten Bilddatensatzes des Patienten ein optimaler Behandlungsort für den Patienten, d.h. ein bevorzugter Zielort für das Fahrzeug, bestimmt werden kann, so dass eine darauf abgestimmte optimale Behandlung des Patienten am Behandlungsort gewährleistet werden kann. Optimal kann dabei insbesondere den Behandlungserfolg jedoch auch Parameter wie die Kosteneffizienz berücksichtigen. Dabei soll vorzugsweise die Notwendigkeit einer Übertragung des Bilddatensatzes an eine zentrale Einheit, d.h. beispielsweise an ein Krankenhaus, für eine erste Bewertung und Diagnose basierend auf dem Bilddatensatz vermieden werden, so dass keine zeitliche Verzögerung in Kauf genommen werden muss, und der Patient möglichst zeitnah den geeigneten Behandlungsort erreicht.

Das medizinisches Bildgebungsgerät kann beispielsweise ein Bildgebungsgerät basierend auf Röntgenstrahlung, beispielsweise ein CT-Gerät, ein C-Bogenröntgengerät oder ein Angiographie-Röntgengerät umfassen. Es sind daneben jedoch auch andere medizinische Bildgebungsgeräte denkbar, welche ausgebildet sind einen zweidimensionalen oder auch dreidimensionalen Bilddatensatz des Patienten zu erzeugen.

Das Bereitstellen des Bilddatensatzes kann beispielsweise ein Erfassen und/oder Auslesen eines computerlesbaren Datenspeichers und/oder ein Empfangen aus einer Speichereinheit mittels der ersten Schnittstelle umfassen. Ferner kann das erfindungsgemäße Verfahren auch das Erzeugen des Bilddatensatz mittels des medizinischen Bildgebungsgeräts umfassen, welcher anschließend für die weiteren Schritte des Verfahrens mittels der ersten Schnittstelle bereitgestellt werden kann.

Der Bilddatensatz kann insbesondere ein dreidimensionaler Bilddatensatz (3D Bilddatensatz) oder ein zweidimensionaler Bilddatensatz (2D Bilddatensatz) sein. Ein 2D Bilddatensatz erlaubt eine zweidimensionale, insbesondere räumlich zweidimensionale, Darstellung eines Untersuchungsbereichs des Patienten. Ein 3D Bilddatensatz erlaubt insbesondere eine dreidimensionale, insbesondere räumlich dreidimensionale, Darstellung eines Untersuchungsbereichs des Patienten. Ein dreidimensionaler Bilddatensatz kann auch als eine Anzahl an Schichtbilddatensätzen dargestellt werden. Ein Schichtbilddatensatz umfasst jeweils eine Schicht des 3D Bilddatensatz an einer Position entlang einer ausgezeichneten Achse. Ein Schichtbilddatensatz erlaubt dann jeweils eine zweidimensionale, insbesondere räumlich zweidimensionale, Darstellung der jeweiligen Schicht des 3D Bilddatensatzes. Vorteilhafterweise umfasst ein 3D Bilddatensatz mehrere Voxel, insbesondere Bildpunkte. Dabei kann jedes Voxel vorzugsweise jeweils einen Wert, insbesondere einen Bildwert, aufweisen, beispielsweise einen Grauwert und/oder einen RGB-Farbwert und/oder einen Intensitätswert. Analog dazu kann ein zweidimensionaler Bilddatensatz mehrere Pixel, insbesondere Bildpunkte, umfassen. Dabei kann jeder Pixel vorzugsweise jeweils einen Wert, insbesondere einen Bildwert, aufweisen, beispielsweise einen Grauwert und/oder einen RGB-Farbwert und/oder einen Intensitätswert.

Der Bilddatensatz kann beispielsweise ein Angiographie-Bilddatensatz, ein Bilddatensatz ohne Kontrastmittelunterstützung oder auch ein Perfusions-Bilddatensatz sein.

Der Patient kann ein menschlicher Patient und/oder ein tierischer Patient sein. Ein vom Bilddatensatz umfasster, d.h. dargestellter, Untersuchungsbereich des Patienten kann einen anatomischen und/oder räumlichen Bereich des Patienten umfassen, der einen vorbestimmten Gewebebereich und/oder einen für eine Diagnose notwendigen räumlichen Bereich umfasst. Der Untersuchungsbereich kann dabei einen Körperbereich, beispielsweise den Kopf oder den Thorax, des Patienten umfassen. Der Untersuchungsbereich kann gegebenenfalls auch den gesamten Körper des Patienten umfassen. Beispielsweise ist der Patient ein Schlaganfallpatient, was einem Patienten aufweisend einen Schlaganfall, d.h. insbesondere einen ischämischen Infarkt, aber auch einem Patienten mit zumindest dem Verdacht auf einen Schlaganfall entsprechen kann. Der Untersuchungsbereich kann dann zumindest einen Teilbereich des Kopfs des Schlaganfallpatienten umfassen.

Das Bewerten basiert insbesondere auf einer Analyse des ersten Bilddatensatzes mittels der Recheneinheit. Die Analyse kann auf den Bildwerten oder durch sie abgebildeten Strukturen, beispielsweise Kanten, zusammenhängende Gruppen von Bildwerten, Schwankungen zwischen benachbarten Bildwerten oder Veränderungen von Bildwerten über die Zeit oder ähnlichem, basieren. Das Bewerten bzw. die Analyse kann ein Schwellwertverfahren, ein Segmentationsverfahren oder ähnliches umfassen. Das Bewerten bzw. die Analyse kann dabei einen Vergleich mit erwarteten Bildwerten oder erwarteten Strukturen einbeziehen, woraus eine Bewertung bzw. ein Analyseergebnis abgeleitet werden kann. Erwartungen können beispielsweise auf dem Bilddatensatz selbst, etwa auf einem Vergleich unterschiedlicher Bereiche des Bilddatensatzes, auf einem anatomischen Atlas bzw. Modell oder auch auf mehreren ähnlichen, bereits vorhandenen Bilddatensätzen des Patienten oder anderer Patienten basieren. Beispielsweise kann eine trainierte Funktion für das Bewerten oder zumindest Teilaspekte des Bewertens eingesetzt werden.

Das Bewerten des bereitgestellten Bilddatensatzes mittels der Recheneinheit kann umfassen den Bilddatensatz zumindest hinsichtlich der Eingruppierung in zumindest eine Bewertungsgruppe zu klassifizieren. Das heißt, der bereitgestellte Bilddatensatz kann den Bilddatensatz dahingehend prüfen, ob der Bilddatensatz der zumindest einen Bewertungsgruppe zugeordnet werden kann. Die Eingruppierung kann dabei auf der Analyse des ersten Bilddatensatzes basieren. Das Bewerten des bereitgestellten Bilddatensatzes mittels der Recheneinheit kann auch umfassen den Bilddatensatz in mehrere, verschiedene Kategorien, d.h. Bewertungsgruppen, einzustufen. Der bewertete Bilddatensatz bzw. eine Bewertungsgruppe kann beispielsweise mit einer möglichen Diagnose, einem notwendigen Behandlungsschritt oder einer für eine Behandlung des Patienten notwendigen medizinischen Ausstattung verknüpft sein. Darüber hinaus kann es auch andere Verknüpfungen geben. Eine Diagnose kann das Vorliegen eines Gefäßverschlusses, oder das Schwere oder das Ausmaß eines Gefäßverschlusses, das Vorliegen einer Gehirnblutung oder auch anderweitiges sein. Beispielsweise umfasst die Bewertung, den Bilddatensatz auf das Vorhandensein eines Gefäßverschlusses, insbesondere auf das Vorhandensein einer LVO, zu prüfen. Die Bewertung kann auch umfassen die Bewertung, den Bilddatensatz auf das Vorhandensein einer Gehirnblutung oder einer anderweitigen Beeinträchtigung zu prüfen. Die Bewertung kann außerdem umfassen, den Gefäßverschluss oder die Gehirnblutung im ersten Bilddatensatz zu lokalisieren oder die Art eines von einem Gefäßverschluss betroffenen Blutgefäßes zu bestimmen. Ein bewertete Bilddatensatz bzw. eine Bewertungsgruppe kann insbesondere mit zumindest einem Kriterium verknüpft sein, welches für eine optimale Behandlung des Patienten an einem Behandlungsort erfüllt sein muss.

Das Bewerten kann vorzugsweise automatisch und ohne weiters Zutun eines Bedieners oder zumindest teil-automatisch basierend auf dem bereitgestellten Bilddatensatz mittels der Recheneinheit durchgeführt werden. Beispielsweise kann ein Starten oder Stoppen des erfindungsgemäßen Verfahrens oder eine Bestätigung einer Eingruppierung, oder ein Triggern weiterer Schritte basierend auf der Bewertung durch einen Bediener vorgesehen sein.

Die Position des Fahrzeugs kann im Schritt des ersten Bestimmens mittels einer Positionsbestimmungseinheit bestimmt werden oder von diesem abgefragt werden, beispielsweise einem satellitengestützten Positionsbestimmungssystems wie etwa GPS oder das europäische globale Satellitennavigationssystem Galileo. Das Fahrzeug kann dann beispielweise in Kombination mit Kartendaten auf einer Karte verortet werden. Dazu kann das Fahrzeug eine Positionsbestimmungseinheit, beispielsweise in Form eines GPS-Empfängers, aufweisen, welcher eine Positionsbestimmung des Fahrzeugs ermöglicht. Die Positionsbestimmungseinheit kann ein Abfragen der Position des Fahrzeugs mittels der Recheneinheit für den Schritt des ersten Bestimmens erlauben. Beispielsweise umfasst das Fahrzeug ein Navigationssystem umfassend eine Positionsbestimmungseinheit, wobei das Bestimmen der Position des Fahrzeugs mittels der Recheneinheit die Abfrage der Position von dem Navigationssystem umfasst.

Das zweite Bestimmen umfasst das Bestimmen einer Anzahl an optionalen Zielorten. Ein optionaler Zielort für das Fahrzeug ist insbesondere von den möglichen, mittels des Fahrzeugs erreichbaren, d.h. ansteuerbaren, Behandlungsorten für den Patienten umfasst. Ein Behandlungsort kann im Wesentlichen ein Krankenhaus oder eine anderweitig für die Behandlung von Patienten, insbesondere Notfallpatienten, ausgelegtes Patientenversorgungszentrum sein. Bei einer Einschränkung des Verfahrens auf Schlaganfallpatienten können die Behandlungsorte beispielsweise lediglich Schlaganfallzentren umfassen. Diese können insbesondere für die Behandlung von Schlaganfallpatienten ausgelegte Krankenhäuser entsprechen. Ein jeweiliger Behandlungsort und damit auch ein jeweiliger Zielort kann beispielsweise durch dessen Ortsinformation lokalisierbar sein. Die Ortsinformation kann beispielsweise in Form von Ortskoordinaten, etwa GPS-Koordinaten, oder auch dessen Adresse vorliegen. Die Anzahl an optionalen Zielorten, welche im Schritt des zweiten Bestimmens bestimmt werden, kann eine Auswahl an Behandlungsorten darstellen. Die optionalen Zielorte sind insbesondere von den Behandlungsorten umfasst und stellen zumindest eine Teilmenge der Behandlungsorte dar.

Die bestimmte Anzahl an optionalen Zielorten wird zumindest basierend auf der bestimmten Position des Fahrzeugs bestimmt. Beispielsweise umfasst die Anzahl an optionalen Zielorte diejenigen Behandlungsorte, welche in einem bestimmten Umkreis, innerhalb einer bestimmten Fahrtstrecke oder innerhalb einer bestimmten Fahrtdauer ausgehend von der Position des Fahrzeugs liegen. Methoden ein Ziel innerhalb eines Umkreises, eine Route oder Fahrtstrecke zu einem bestimmten Ziel oder eine Vorhersage einer Fahrtdauer, um das Ziel zu erreichen, zu ermitteln, sind im Feld der Navigationssysteme breit bekannt und werden daher hier nicht weiter ausgeführt.

Die Anzahl an optionalen Zielorten kann auch anhand weiterer Kriterien, beispielsweise einer Verfügbarkeit der medizinischen Ausstattung an einem Behandlungsort oder eines Ausstattungslevels, bestimmt werden. Beispielsweise umfasst die Anzahl an optionalen Zielorten eine Anzahl an für eine Behandlung des Patienten momentan verfügbaren Behandlungsorten oder eine Anzahl an Behandlungsorten mit einem bestimmten Ausstattungslevel. Beispielsweise umfasst die Anzahl an optionalen Zielorten eine Anzahl an CSCs/TCSCs und/oder eine Anzahl an PSCs welche innerhalb eines bestimmten Umkreises um die Position des Fahrzeugs lokalisiert sind.

Für die Bestimmung der Anzahl an optionalen Zielorten kann eine lokale Datenbank von Behandlungsorten, beispielweise in Form einer Tabelle oder Liste mit der jeweiligen Ortsinformation auf einer Speichereinheit der Recheneinheit oder des Fahrzeugs lokal vorliegen, welche für den Schritt des zweiten Bestimmens für die Recheneinheit abrufbar und verarbeitbar bereitgestellt ist. Mit jedem Behandlungsort können außerdem auch weitere Informationen, beispielsweise ein Ausstattungslevel oder ähnliches, verknüpft und auf der Datenbank abrufbar vorgehalten werden. Das zweite Bestimmen der Anzahl an optionalen Zielorten kann alternativ oder zusätzlich auch eine Abfrage von einer zentralen Einheit umfassen. Die zentrale Einheit kann an einem Krankenhaus oder auch an anderer Stelle, beispielsweise einem Computerzentrum, lokalisiert sein.

Die zentrale Einheit kann ebenfalls eine Datenbank an Behandlungsorten vorhalten, welche beispielsweise über ein Funknetz, insbesondere Mobilfunknetz, abgefragt werden kann. Die zentrale Einheit kann zusätzliche Informationen über Behandlungsorte abrufbar vorhalten. Insbesondere kann die zentrale Einheit weitere mit den Behandlungsorten verknüpfte Informationen bereitstellen, welche auf der zentralen Einheit insbesondere auch zeitlich aktualisierbar vorgehalten werden. Beispielweise kann mittels der zentralen Einheit die aktuelle Verfügbarkeit der medizinischen Ausstattung an einem Behandlungsort abgefragt werden. Das Fahrzeug oder die Recheneinheit kann dafür insbesondere eine Kommunikationseinheit vorsehen, welche ausgebildet ist, eine Abfrage, insbesondere über ein Funknetz, von einer zentralen Einheit durchzuführen.

Aus der Anzahl an optionalen Zielorten wird der bevorzugte Zielort mittels der Recheneinheit basierend auf dem bewerteten Bilddatensatz bestimmt oder ausgewählt.

Der bevorzugte Zielort kann anhand von zumindest einem Kriterium bestimmt werden. Der bewertete Bilddatensatz kann mit zumindest einem Kriterium verknüpft sein, welche von einem optionalen Zielort für die Behandlung des Patienten bevorzugt erfüllt sein muss. Das zumindest eine Kriterium kann mit den bestimmten optionalen Zielorten abgeglichen werden. Basierend auf dem Abgleich kann der bevorzugte Zielort bestimmt werden. Je nach Bewertung kann ein unterschiedlicher optionaler Zielort als bevorzugter Zielort bestimmt werden. Ist die Bewertung des Bilddatensatzes beispielsweise mit einer möglichen Diagnose, einem notwendigen Behandlungsschritt oder mit für eine Behandlung des Patienten notwendigen medizinischen Ausstattung verknüpft, kann daraus direkt eine notwendige medizinische Ausstattung als Kriterium abgelesen oder aus dieser abgeleitet werden. Dieses Kriterium kann dann mit den bestimmten optionalen Zielorten abgeglichen werden, worauf basierend der bevorzugte Zielort bestimmt werden kann. Ein weiteres Kriterium kann eine möglichst zeitnahe Erreichbarkeit des Zielorts mit dem Fahrzeug darstellen. Die Erreichbarkeit kann anhand einer Fahrtstrecke oder anhand einer vorhergesagten Fahrtdauer, welche anhand der Position des Fahrzeugs und den optionalen Zielorten bestimmt werden kann, ermittelt werden. Ein weiteres Kriterium kann beispielsweise die Verfügbarkeit der medizinischen Ausstattung sein. Es sind auch weitere Kriterien für einen Abgleich denkbar.

Das Ausgeben des ausgewählten bevorzugten Zielorts kann umfassen, dass der Zielort mittels der zweiten Schnittstelle an eine Ausgabeeinheit ausgegeben wird. Die Ausgabeeinheit kann ausgebildet sein, den Zielort für einen Fahrer des Fahrzeugs visuell oder akustisch auszugeben bzw. wahrnehmbar bereitzustellen. Die Ausgabeeinheit kann beispielsweise ein Display oder ein Lautsprecher umfassen. Das Ausgeben des ausgewählten bevorzugten Zielorts kann umfassen, dass der Zielort mittels der zweiten Schnittstelle an ein vom Fahrzeug oder von der Recheneinheit umfassten Navigationssystem ausgegeben wird.

Der bevorzugte Zielort kann etwa in Form einer Ortsinformation, d.h. einer Adresse oder in Form von Ortskoordinaten oder auch anderweitig ausgegeben werden.

Die Erfinder haben erkannt, dass vorteilhaft eine besonders zeiteffiziente und optimierte Behandlung eines Patienten durch das erfindungsgemäße Verfahren gewährleistet werden kann, indem basierend auf dem ersten Bilddatensatz mittels einer lokal im Fahrzeug vorgehaltenen Recheneinheit eine Bewertung durchgeführt wird und ein darauf abgestimmter optimaler Behandlungsort als bevorzugter Zielort für das Fahrzeug bestimmt wird. Vorteilhaft kann eine zeitliche Verzögerung durch eine Übertragung des Bilddatensatzes für eine telemedizinische Bewertung vermieden werden. Vorteilhaft kann stets ein Behandlungsort gewählt werden, welcher hinsichtlich der Erreichbarkeit, der bereitgestellten Ausstattung und der Kosteneffizienz optimal für die Behandlung des Patienten ist.

In einer bevorzugten Variante des Verfahrens wird im Schritt des zweiten Bestimmens außerdem zumindest ein Parameter der folgenden Liste bestimmt
- Medizinische Ausstattung an einem jeweiligen optionalen Zielort für die Behandlung des Patienten,
- Verfügbarkeit der medizinischen Ausstattung an einem jeweiligen optionalen Zielort für die Behandlung des Patienten,
- Fahrroute zu einem jeweiligen optionalen Zielort,
- Fahrtdauer zu einem jeweiligen optionalen Zielort.

Der zumindest eine bestimmte Parameter kann in den Schritt des zweiten Bestimmens der optionalen Zielorte und/oder in den Schritt des dritten Bestimmens des bevorzugten Zielorts eingehen. Das kann bedeuten, dass die Anzahl an optionalen Zielorten basierend auf zumindest einem dieser Parameter eingeschränkt werden kann. Bevorzugt geht der zumindest eine Parameter als Kriterium für die Bestimmung des bevorzugten Zielorts aus der Anzahl an optionalen Zielorten mit ein.

Die Medizinische Ausstattung kann beispielweise durch eine Einstufung des Behandlungsort bzw. des optionalen Zielorts anhand bestimmter Ausstattungslevel widergegeben sein. Diese Information kann gemeinsam mit der Ortsinformation der Behandlungsorte für die Recheneinheit abrufbar bereitgestellt werden kann. Es können beispielsweise zumindest CSCs/TCSCs von PSCs unterschieden werden. Es kann auch andere Unterscheidungen oder Einstufungen geben. Die medizinische Ausstattung der Behandlungsorte kann auch detaillierter bereitgestellt sein und beispielsweise jeweils die unterschiedlichen Behandlungsmöglichkeiten in Form der vorgehaltenen medizinischen Geräte und/oder des am Behandlungsort anwesenden, für eine Behandlung geschulten medizinischen Personals als abrufbare Information wiedergeben.

Die Verfügbarkeit der medizinischen Ausstattung kann eine Auslastung der Behandlungsmöglichen, d.h. der medizinischen Geräte und/oder des medizinischen Personals an einem jeweiligen Behandlungsort umfassen. Die Verfügbarkeit kann insbesondere als zeitlich aktualisierte Information abgefragt werden. Beispielsweise können nur verfügbare Behandlungsorte als optionale Zielorte im Schritt des zweiten Bestimmens bestimmt werden. Beispielsweise kann nur ein verfügbarer optionaler Zielort als bevorzugter Zielort im Schritt des dritten Bestimmens bestimmt werden.

Insbesondere kann der zumindest eine Parameter automatisch mittels der Recheneinheit bestimmt werden. Der zumindest eine Parameter kann anhand einer lokal vorgehaltenen Datenbank, welche einen Behandlungsort jeweils mit dem zumindest einen Parameter verknüpft vorhält, abgefragt werden. Es kann auch eine Abfrage einer zentral vorgehaltenen Datenbank an einer zentralen Einheit umfasst sein.

Durch Bestimmen einer Fahrroute zu einem jeweiligen Behandlungsort bzw. zu einem jeweiligen optionalen Zielort kann Rückschlüsse über eine Fahrtstrecke ermöglichen. Eine bestimmte Fahrroute kann außerdem auch Rückschlüsse über eine vorhergesagte Fahrtdauer ermöglichen. Eine vorhergesagte Fahrtdauer kann auch ein momentanes oder vorhergesagtes Verkehrsaufkommen auf der Fahrtroute miteinbeziehen. Beispielsweise können lediglich optionale Zielorte innerhalb einer bestimmten Fahrtstrecke oder Fahrtdauer bestimmt werden. Beispielsweise kann der bevorzugte Zielort basierend auf der kürzesten Fahrtstrecke oder der kürzesten Fahrtdauer bestimmt werden. Die Fahrroute oder die vorhergesagte Fahrdauer kann mittels eines vom Fahrzeug oder von der Recheneinheit umfassten Navigationssystems bestimmt werden oder von einem solchen von der Recheneinheit für das Bestimmen abgefragt werden. Methoden eine Fahrtroute oder darauf basierend eine Fahrdauer zu bestimmen bzw. Vorherzusagen sind dabei in dem Bereich der Navigationssysteme weit verbreitet und sollen hier nicht im Detail ausgeführt werden.

Die Parameter können für die Bestimmung des bevorzugten Zielorts insbesondere abgestimmt auf den bewerteten Bilddatensatz kombiniert werden. Beispielsweise kann je nach Bewertung des Bilddatensatzes derjenige verfügbare optionale Zielort mit einem notwendigen Ausstattungslevel als bevorzugter Zielort gewählt werden, welcher in kürzest möglicher Zeit mittels des Fahrzeugs erreichbar ist.

Vorteilhaft erlaubt die Bestimmung zumindest eines der obigen Parameter eine besonders vorteilhafte Bestimmung der optionalen Zielorte und insbesondere die Auswahl des bevorzugten Zielorts mittels der Recheneinheit, so dass eine optimale, zeiteffiziente Behandlung möglich ist.

In einer weiteren vorteilhaften Verfahrensvariante umfasst der Schritt des Bewertens das Prüfen des bereitgestellten Bilddatensatzes auf das Vorliegen eines Gefäßverschlusses eines Blutgefäßes des Patienten.

Insbesondere kann der Schritt des Bewertens das Prüfen des bereitgestellten Bilddatensatzes auf das Vorliegen eines Gefäßverschlusses eines Blutgefäßes im Gehirn des Patienten umfassen. Das Vorliegen eines Gefäßverschlusses, insbesondere im Gehirn des Patienten, ist insbesondere von dem Erfordernis einer besonders zeitnahen und optimalen Behandlung eines Patienten begleitet, so dass sich hier die Vorteile des erfindungsgemäßen Verfahrens besonders vorteilhaft zu einem Behandlungserfolg beitragen können.

Das Prüfen im Schritt des Bewertens kann ein Feststellen eines Vorhandenseins eines Gefäßverschlusses im Untersuchungsbereich umfassen. Das heißt, ein bewerteter Bilddatensatz kann lediglich dahingehend bewertet sein, ob ein Gefäßverschluss im Untersuchungsbereich vorliegt. Das Vorliegen eines Gefäßverschlusses kann beispielsweise mit einer Bewertungsgruppe verknüpft sein oder dieser entsprechen, in welche der Bilddatensatz im Schritt des Bewertens eingruppiert bzw. klassifiziert werden kann.

Das Prüfen kann auch ein Lokalisieren des Gefäßverschlusses umfassen. Das heißt, ein bewerteter Bilddatensatz kann dahingehend bewertet sein, ob ein Gefäßverschluss vorliegt und wo im ersten Bilddatensatz oder im Untersuchungsbereich ein Gefäßverschluss vorliegt. Der Ort des Gefäßverschlusses kann dabei einen Hinweis auf die Auswirkungen des Gefäßverschlusses oder auf notwendige Behandlungsschritte ermöglichen und damit wesentliche Hinweise für die Bewertung bzw. eine Eingruppierung in eine entsprechende Bewertungsgruppe und damit auch für eine optimale Behandlung liefern. Der Ort eines Gefäßverschlusses kann beispielsweise mit einer Bewertungsgruppe verknüpft sein, in welche der Bilddatensatz im Schritt des Bewertens eingruppiert bzw. klassifiziert werden kann.

Das Prüfen kann dabei auch umfassen, das Blutgefäß oder die Art des Blutgefäßes zu bestimmen, in welchem der Gefäßverschluss vorliegt. Beispielsweise kann für die Bewertung zwischen unterschiedlichen Blutgefäßen unterschieden werden. Die unterschiedlichen Blutgefäße bzw. Blutgefäßarten können beispielsweise eine unterschiedlich wichtige Rolle in der Blutversorgung des Gehirns des Patienten spielen. Die Blutgefäßart, in welchem ein Gefäßverschluss vorliegt, kann beispielsweise mit einer Bewertungsgruppe verknüpft sein, in welche der Bilddatensatz im Schritt des Bewertens eingruppiert bzw. klassifiziert werden kann.

Unterschiedliche Orte oder Blutgefäßarten können mit unterschiedlichen Erfordernissen und Notwendigkeiten für die Behandlung eines Patienten verknüpft sein, so dass ein Eingehen einer derartigen Information in das Bewerten und ein Bestimmen eines bevorzugten Zielortes darauf basierend vorteilhaft zu einer Optimierung des Behandlungsablaufs führen kann.

In einer vorteilhaften Verfahrensvariante wird bei Vorliegen eines Gefäßverschlusses außerdem ein Ort des Gefäßverschlusses oder eine Art des vom Gefäßverschluss betroffenen Blutgefäß bestimmt wird. Beispielsweise kann eine LVO, d.h. ein Gefäßverschluss einer großen Gehirnarterie, bestimmt werden.

Beispielsweise kann eine LVO mit einer Bewertungsgruppe verknüpft sein, in welche der Bilddatensatz im Schritt des Bewertens eingruppiert bzw. klassifiziert werden kann.

Je nach Bewertung können unterschiedliche Ausstattungslevel d.h. unterschiedliche Zielorte für die Behandlung des Patienten notwendig sein. So kann insbesondere bei einer LVO ein CSC/TCSC-Level eines Schlaganfallzentrums notwendig für die Behandlung eines Patienten sein, wohingegen bei einem Gefäßverschluss eines kleinen, nebengeordneten Blutgefäßes ein PSC ausreichen sein kann.

Dabei kann in einer Ausführung des Verfahrens im Schritt des Bewertens ein maschinelles Lernverfahren/einer trainierten Funktion angewendet werden.

Durch Anwendung der trainierten Funktion auf den bereitgestellten Bilddatensatz kann eine Bewertung des Bilddatensatzes ermöglicht werden. Die trainierte Funktion kann auch auf einen Teilaspekt des Bewertens, beispielsweise das Prüfen, Lokalisieren und/oder Einstufen bzw. Eingruppieren, eingeschränkt sein.

Dabei kann die trainierte Funktion vorteilhafterweise durch ein Verfahren des maschinellen Lernens trainiert sein. Insbesondere kann die trainierte Funktion ein neuronales Netzwerk, insbesondere ein faltendes neuronales Netzwerk (engl. convolutional neural network, CNN) bzw. ein Netzwerk umfassend eine Faltungsschicht (engl. convolutional layer) sein.

Eine trainierte Funktion bildet Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Ausgabedaten auf die Trainingseingabedaten angewendet wird. Insbesondere kann das Bewerten auf einem Vergleich der erzeugten Ausgabedaten und der Trainingsausgabedaten basieren. Im Allgemeinen wird auch eine trainierbare Funktion, d.h. eine Funktion mit noch nicht angepassten einen oder mehreren Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk, wobei die Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (engl. deep neural network, deep artificial neural network). Ein weiteres Beispiel für eine trainierte Funktion ist eine "Support Vector Machine", weiterhin sind auch insbesondere andere Algorithmen des maschinellen Lernens als trainierte Funktion einsetzbar. Die trainierte Funktion kann beispielsweise mittels einer Rückpropagation trainiert sein. Zunächst können Ausgabedaten durch Anwendung der trainierten Funktion auf Trainingseingabedaten erzeugt werden. Hiernach kann eine Abweichung zwischen den erzeugten Ausgabedaten und den Trainingsausgabedaten durch Anwendung einer Fehlerfunktion auf die erzeugten Ausgabedaten und die Trainingsausgabedaten ermittelt werden. Ferner kann zumindest ein Parameter, insbesondere eine Gewichtung, der trainierten Funktion, insbesondere des neuronalen Netzwerks, basierend auf einem Gradienten der Fehlerfunktion bezüglich des zumindest einen Parameters der trainierten Funktion iterativ angepasst werden. Hierdurch kann die Abweichung zwischen den Trainingsabbildungsdaten und den Trainingsausgabedaten während des Trainings der trainierten Funktion vorteilhafterweise minimiert werden.

Die Eingabedaten können eine Vielzahl an mittels des medizinischen Geräts erzeugten Bilddatensätze umfassen. Die Trainingsausgabedaten können korrespondierende bewertete Bilddatensätze umfassen.

Durch Anwenden eines künstlichen Intelligenzsystems, d.h. einer trainierten Funktion, können alle relevanten Einflussgrößen für das Bewerten berücksichtigt werden, auch solche, für die ein Anwender keinen Zusammenhang zum Bewerten abschätzen kann. Insbesondere kann mittels einer trainierten Funktion nach der Trainingsphase eine Bewertung besonders verlässlich und zeiteffizient automatisiert ermöglicht werden. Vorteilhaft kann eine telemedizinische Bewertung und eine damit einhergehende Zeitverzögerung vermieden werden.

Weiterhin kann das erfindungsgemäße Verfahren umfassen, dass der erzeugte Bilddatensatz ein CT-Angiographie-Bilddatensatz ist.

Ein CT-Angiographie -Bilddatensatz erlaubt besonders vorteilhaft eine Bewertung hinsichtlich eines Gefäßverschlusses eines Blutgefäßes eines Patienten, insbesondere eines Schlaganfallpatienten.

In einer Ausführungsvariante des erfindungsgemäßen Verfahrens wird der Schritt des ersten Bestimmens und der Schritt des zweiten Bestimmens wiederholt ausgeführt, so dass bei einer aktualisierten Position des Fahrzeugs eine aktualisierte Anzahl an optionalen Zielorten bestimmt wird.

Vorteilhaft kann auch bei einer aktualisierten Position des Fahrzeugs, d.h. wenn das Fahrzeug sich bewegt hat, ein optimaler Behandlungsort, d.h. ein optimaler Zielort, bestimmt werden. Beispielsweise kann stets eine aktuelle Verfügbarkeit oder eine aktuelle vorhergesagte Fahrtdauer in das dritte Bestimmen des bevorzugten Zielorts miteingehen.

Weiterhin kann vorgesehen sein, dass der Schritt des ersten Bestimmens und/oder der Schritt des zweiten Bestimmens während des Schritts des Bewertens durchgeführt wird.

Während kann bedeuten, dass die Schritte im Wesentlichen zeitgleich durchgeführt werden oder zumindest in zeitlicher Nähe zueinander durchgeführt, gestartet oder beendet werden. Vorteilhaft kann zeitgleich oder zumindest in kurzer zeitlicher Nähe mit dem Abschluss des Schritts des Bewertens, d.h. bei Vorhandensein einer Bewertung, die Anzahl an optionalen Zielorten bestimmt sein und für den Schritt des dritten Bestimmens des bevorzugten Zielorts zur Verfügung stehen. Vorteilhaft kann ein zeiteffizienter Ablauf gewährleistet werden.

In einer weiteren Variante des erfindungsgemäßen Verfahrens werden basierend auf dem bestimmten bevorzugten Zielort Navigationsinformationen zur Erreichung des bevorzugten Zielorts ermittelt und mittels einer Ausgabeeinheit ausgegeben.

Beispielsweise umfasst die Recheneinheit oder das Fahrzeug ein Navigationssystem, um Navigationsinformationen für den Fahrer des Fahrzeugs zum Erreichen eines Navigationsziels zu ermitteln. Dabei kann der bevorzugte Zielort automatisch mittels der Schnittstelle als ausgewähltes Navigationsziel in das Navigationssystem programmiert werden kann. Der Fahrer kann dann anhand von dem Navigationssystem bereitgestellten Navigationsinformationen zu dem bevorzugten Zielort geleitet werden. Die Navigationsinformation kann visuelle und/oder akustische Information umfassen. Beispielsweise umfasst die Navigationsinformation Kartendaten, auf welche der Zielort und/oder eine Route zu dem Zielort ausgehend von der aktuellen Position des Fahrzeugs anzeigt wird, oder Sprachanweisungen. Die Ausgabeeinheit kann dann entsprechend beispielsweise ein Display zur Darstellung der Navigationsinformation für den Fahrer oder ein Lautsprecher für die akustische Ausgabe der Navigationsinformation an den Fahrer umfassen.

Die Erfindung betrifft weiterhin ein Fahrzeug, insbesondere Krankenwagen, umfassend
- ein medizinisches Bildgebungsgerät, ausgebildet einen Bilddatensatz eines Patienten zu erzeugen,
- eine erste Schnittstelle, ausgebildet den erzeugten Bilddatensatz für eine Weiterverarbeitung bereitzustellen,
- eine Recheneinheit, ausgebildet
   - den erzeugten Bilddatensatz zu bewerten,
   - eine Position des Fahrzeugs zu bestimmen,
   - eine Anzahl an optionalen Zielorten für eine Behandlung des Patienten zumindest basierend auf der bestimmten Position des Fahrzeugs zu bestimmen,
   - einen bevorzugten Zielort aus der Anzahl an optionalen Zielorten zumindest basierend auf dem bewerteten Bilddatensatz und der bestimmten Position des Fahrzeugs auszuwählen,
- eine zweite Schnittstelle, ausgebildet den ausgewählten, bevorzugten Zielort auszugeben.

Das Fahrzeug kann beispielsweise eine sogenannte Mobile Stroke Unit sein. Ein erfindungsgemäßes Fahrzeug kann insbesondere dazu ausgebildet sein die zuvor beschriebenen erfindungsgemäßen Verfahren und ihre Aspekte auszuführen. Das erfindungsgemäße Fahrzeug kann dazu ausgebildet sein die Verfahren und ihre Aspekte auszuführen, indem das medizinische Bildgebungsgerät, die Recheneinheit und die Schnittstellen ausgebildet sind, die entsprechenden Verfahrensschritte auszuführen.

Die Ausgestaltungsvarianten, Merkmale und Vorteile des Verfahrens und seiner Ausführungsformen können dabei ebenso direkt auf das erfindungsgemäße Fahrzeug übertragen werden.

In einer bevorzugten Vorrichtungsvariante ist das medizinische Bildgebungsgerät insbesondere ein CT-Gerät. Das CT-Gerät kann dabei insbesondere ausgebildet ein neben nativen CT-Bilddatensätzen, CT-Angiographie-Bilddatensätze und Perfusions-CT-Bilddatensätze zu erzeugen.

Die Erfindung betrifft außerdem ein Computerprogrammprodukt mit einem Computerprogramm, welches direkt in eine Speichereinheit einer Recheneinheit eines Fahrzeugs, insbesondere eines Krankenwagens, ladbar ist, mit Programmabschnitten, um alle Schritte des Verfahrens zum Bestimmen eines bevorzugten Zielorts für ein Fahrzeug auszuführen, wenn die Programmabschnitte von der Recheneinheit ausgeführt werden.

Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Recheneinheit zu laden ist. Durch das Computerprogrammprodukt kann das Verfahren zur Ansteuerung eines medizinischen Bildgebungsgeräts schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer Datenverarbeitungseinheit geladen werden kann, der mit der Datenverarbeitungseinheit direkt verbunden oder als Teil der Datenverarbeitungseinheit ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Verarbeitungseinheit ein erfindungsgemäßes Verfahren durchführen. Beispiele für elektronisch lesbare Datenträger sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Verarbeitungseinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Eine weitgehend softwaremäßige Realisierung hat den Vorteil, dass auch schon bisher verwendete Vorrichtungen zur Ansteuerung eines medizinischen Bildgebungsgeräts auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile, wie z.B. eine Dokumentation und/oder zusätzliche Komponenten, sowie Hardware-Komponenten, wie z.B. Hardware-Schlüssel (Dongles etc.) zur Nutzung der Software, umfassen.

Die Erfindung betrifft außerdem ein Computerprogramm, welches direkt in einen Speichereinheit einer Recheneinheit eines Fahrzeugs, insbesondere eines Krankenwagens, ladbar ist, mit Programmabschnitten, um alle Schritte eines Verfahrens zum Bestimmen eines bevorzugten Zielorts für ein Fahrzeug auszuführen, wenn die Programmabschnitte von der Recheneinheit ausgeführt werden.

Die Erfindung betrifft außerdem ein Computerlesbares Speichermedium, auf welchem von einer Recheneinheit lesbare und ausführbare Programmabschnitte gespeichert sind, um alle Schritte eines Verfahrens zum Bestimmen eines bevorzugten Zielorts für ein Fahrzeug auszuführen, wenn die Programmabschnitte von der Recheneinheit ausgeführt werden.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 einen schematischen Verfahrensablauf eines Verfahrens zum Bestimmen eines bevorzugten Zielorts für ein Fahrzeug,
Fig. 2 einen schematischen Verfahrensablauf einer weiteren Variante eines Verfahrens zum Bestimmen eines bevorzugten Zielorts für ein Fahrzeug,
Fig. 3 schematisch eine Veranschaulichung einer Anzahl an optionalen Zielorten,
Fig. 4 eine schematische Darstellung eines Fahrzeugs umfassend ein medizinisches Bildgebungsgerät.

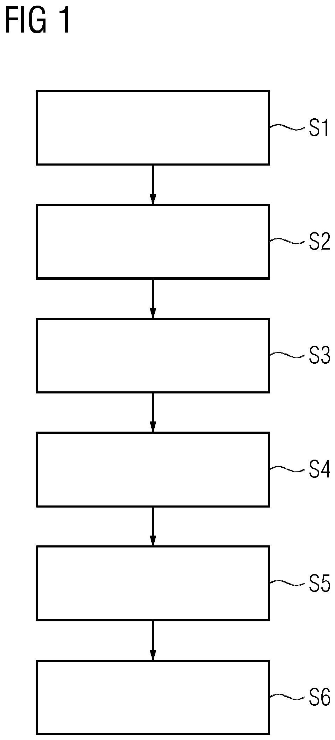

Fig. 1 zeigt einen schematischen Verfahrensablauf eines Verfahrens zum Bestimmen eines bevorzugten Zielorts für ein Fahrzeug 100, insbesondere einen Krankenwagen, aufweisend ein medizinisches Bildgebungsgerät 101. Beispielsweise ist das medizinische Bildgebungsgerät ein CT-Gerät ausgebildet einen dreidimensionalen Bilddatensatz zu erzeugen.

Das Verfahren umfasst den Schritt des Bereitstellens S1 eines Bilddatensatzes eines Patienten 103, welcher mittels des medizinischen Bildgebungsgeräts 101 erzeugt wurde, mittels einer ersten Schnittstelle 105. Der Bilddatensatz kann beispielsweise ein Angiographie-Bilddatensatz, ein Bilddatensatz ohne Kontrastmittelunterstützung oder auch ein Perfusions-Bilddatensatz sein.

Weiterhin wird der bereitgestellte Bilddatensatz in einem Schritt des Bewertens S2 mittels einer Recheneinheit 107 bewertet. Das Bewerten basiert dabei auf einer Analyse des ersten Bilddatensatzes mittels der Recheneinheit 107. Die Analyse kann auf den Bildwerten oder durch sie abgebildeten Strukturen, beispielsweise Kanten, zusammenhängende Gruppen von Bildwerten, Schwankungen zwischen benachbarten Bildwerten oder Veränderungen von Bildwerten über die Zeit oder ähnlichem, basieren. Das Bewerten des bereitgestellten Bilddatensatzes mittels der Recheneinheit 107 kann umfassen den Bilddatensatz zumindest in zumindest eine Bewertungsgruppe zu einzugruppieren. Das heißt, der bereitgestellte Bilddatensatz kann den Bilddatensatz dahingehend prüfen, ob der Bilddatensatz der zumindest einen Bewertungsgruppe zugeordnet werden kann. Ein bewerteter Bilddatensatz bzw. eine Bewertungsgruppe kann insbesondere mit zumindest einem Kriterium verknüpft sein, welches für eine optimale Behandlung des Patienten 103 an einem Behandlungsort erfüllt sein muss. Ein Kriterium kann beispielsweise die medizinische Ausstattung am Behandlungsort, ein bestimmtes Ausstattungslevel am Behandlungsort oder eine Verfügbarkeit des Behandlungsorts betreffen. Das Kriterium kann auch ein anderweitiges Kriterium sein.

Dabei kann der Schritt des Bewertens S2 des Bilddatensatzes umfassen, den Bilddatensatz auf das Vorliegen eines Gefäßverschlusses eines Blutgefäßes des Patienten 103, insbesondere im Gehirn des Patienten 103, zu prüfen. Bei Vorliegen eines Gefäßverschlusses kann dabei außerdem vorzugsweise ein Ort des Gefäßverschlusses oder eine Art des vom Gefäßverschluss betroffenen Blutgefäß bestimmt werden. Beispielsweise umfasst die Bewertung, den Bilddatensatz auf das Vorhandensein eines Gefäßverschlusses, insbesondere auf das Vorhandensein einer LVO, zu prüfen.

Besonders vorteilhaft ist der bereitgestellte Bilddatensatz ein CT-Angiographie-Bilddatensatz. Ein Bewerten des Bilddatensatzes hinsichtlich eines Gefäßverschlusses ist mittels eines CT-Angiographie-Bilddatensatzes besonders vorteilhaft möglich.

Der Schritt des Bewertens S2 umfasst in einer vorteilhaften Variante insbesondere das Anwenden einer trainierten Funktion. Durch Anwenden eines künstlichen Intelligenzsystems, d.h. einer trainierten Funktion, können alle relevanten Einflussgrößen für das Bewerten berücksichtigt werden, auch solche, für die ein Anwender keinen Zusammenhang zum Bewerten abschätzen kann. Insbesondere kann mittels einer trainierten Funktion nach der Trainingsphase eine Bewertung besonders verlässlich und zeiteffizient automatisiert ermöglicht werden.

In einem weiteren Schritt des ersten Bestimmens S3 wird eine Position 4 des Fahrzeugs 100 bestimmt. Die Position 4 des Fahrzeugs 100 kann im Schritt des ersten Bestimmens mittels einer Positionsbestimmungseinheit 113 ermittelt werden oder von dieser abgefragt werden, beispielsweise einem satellitengestützten Positionsbestimmungssystems wie etwa GPS oder das europäische globale Satellitennavigationssystem Galileo. Die Positionsbestimmungseinheit 113 kann dann beispielsweise durch einen GPS Empfänger umgesetzt sein.

In einem weiteren Schritt des zweiten Bestimmens S4 wird eine Anzahl an optionalen Zielorten 1,2,3 für eine Behandlung des Patienten 103 zumindest basierend auf der bestimmten Position des Fahrzeugs mittels der Recheneinheit 107 bestimmt. Die Anzahl an optionalen Zielorte 1,2,3 kann eine Auswahl an Behandlungsorten, d.h. Krankenhäuser, Schlaganfallzentren, oder ähnliches, darstellen. Ein jeweiliger Behandlungsort bzw. Zielort kann dann durch dessen Ortskoordinaten oder dessen Adresse bestimmt sein. Die bestimmte Anzahl an optionalen Zielorte 1,2,3 wird zumindest basierend auf der bestimmten Position des Fahrzeugs bestimmt. Beispielsweise umfasst die Anzahl an optionalen Zielorte 1,2,3 diejenigen Behandlungsorte, welche in einem bestimmten Umkreis, innerhalb einer bestimmten Fahrtstrecke oder innerhalb einer bestimmten Fahrtdauer ausgehend von der Position 4 des Fahrzeugs 100 liegen. Die Anzahl an optionalen Zielorte 1,2,3 kann auch anhand weiterer Kriterien, beispielsweise einer medizinischen Ausstattung oder einer Verfügbarkeit der medizinischen Ausstattung an einem Behandlungsort, eingeschränkt sein.

Anschließend wird in einem Schritt des dritten Bestimmens S5 der bevorzugte Zielort aus der Anzahl an optionalen Zielorten 1,2,3 zumindest basierend auf dem bewerteten Bilddatensatz und der bestimmten Position des Fahrzeugs 100 mittels der Recheneinheit 107 bestimmt. Der bevorzugte Zielort kann basierend auf zumindest einem Kriterium bestimmt werden, wobei das Kriterium insbesondere aus der Bewertung, d.h. dem bewerteten Bilddatensatz, abgeleitet werden kann. Der bewertete Bilddatensatz kann mit zumindest einem Kriterium verknüpft sein, welche von einem optionalen Zielort für die Behandlung des Patienten bevorzugt erfüllt sein muss. Das zumindest eine Kriterium kann mit den bestimmten optionalen Zielorten 1,2,3 abgeglichen werden. Basierend auf dem Abgleich kann der bevorzugte Zielort bestimmt werden.

In einer vorteilhaften Variante wird im Schritt des zweiten Bestimmens S4 außerdem zumindest ein Parameter der folgenden Liste bestimmt
- Medizinische Ausstattung eines jeweiligen optionalen Zielorts 1,2,3 für die Behandlung des Patienten 103,
- Verfügbarkeit der medizinischen Ausstattung eines jeweiligen optionalen Zielorts 1,2,3 für die Behandlung des Patienten 103,
- Fahrroute 5 zu einem jeweiligen optionalen Zielort 1,2,3,
- Fahrtdauer zu einem jeweiligen optionalen Zielort 1,2,3.

Der zumindest eine Parameter kann dabei in den Schritt des zweiten Bestimmens S4 selbst oder auch in den Schritt des dritten Bestimmens S5 des bevorzugten Zielorts basierend auf der Anzahl an optionalen Zielorten 1,2,3 miteingehen. Das kann bedeuten, dass die Anzahl an optionalen Zielorte 1,2,3 basierend auf zumindest einem dieser Parameter eingeschränkt werden kann. Bevorzugt geht der zumindest eine Parameter als Kriterium in das Bestimmen S5 des bevorzugten Zielorts aus der Anzahl an optionalen Zielorten 1,2,3 mit ein.

Beispielsweise umfassen die im Schritt des zweiten Bestimmens bestimmten optionalen Zielorte 1,2,3 sowohl verfügbare Behandlungsorte mit CSC/TCSC-Level als auch mit PSC-Level. Je nach bewerteten Bilddatensatz kann dann entweder ein CSC/ TCSC oder ein PSC im Schritt des dritten Bestimmens S5 als bevorzugter Zielort bestimmt werden. Beispielsweise wird dasjenige CSC/TCSC bzw. dasjenige PSC aufweisend die kürzeste Anfahrt, gemessen in einer Fahrtstrecke oder in einer vorhergesagten Fahrtdauer, als bevorzugter Zielort bestimmt. Beispielsweise wird beim Bewerten des Bilddatensatzes das Vorliegen eines LVO detektiert. Das Vorliegen eines LVO kann mit der Notwendigkeit eines CSC/TCSC-Ausstattungslevel verknüpft sein. Worauf hin das nächstgelegenen CSC/TCSC ausgehend von der Position des Fahrzeugts als bevorzugter Zielort bestimmt wird. Es sind davon abweichend auch andere Szenarien denkbar.

In einem weiteren Schritt des Ausgebens S6 wird der ausgewählte, bevorzugte Zielort mittels einer zweiten Schnittstelle 109 ausgegeben. Das Ausgeben des ausgewählten bevorzugten Zielorts kann umfassen, dass der bevorzugte Zielort mittels der zweiten Schnittstelle 109 an eine Ausgabeeinheit 119 ausgegeben wird. Die Ausgabeeinheit 119 kann ausgebildet sein, den bevorzugten Zielort für einen Fahrer des Fahrzeugs 100 visuell und/oder akustisch auszugeben bzw. wahrnehmbar bereitzustellen. Die Ausgabeeinheit 119 kann beispielsweise ein Display oder ein Lautsprecher umfassen. Das Ausgeben des ausgewählten bevorzugten Zielorts kann auch umfassen, dass der Zielort mittels der zweiten Schnittstelle an ein vom Fahrzeug 100 oder von der Recheneinheit umfassten Navigationssystem 115 ausgegeben wird, welches ausgebildet ist Navigationsinformationen zum Erreichen des bevorzugten Zielorts zu ermitteln und visuell und/oder akustisch für den Fahrer bereitzustellen.

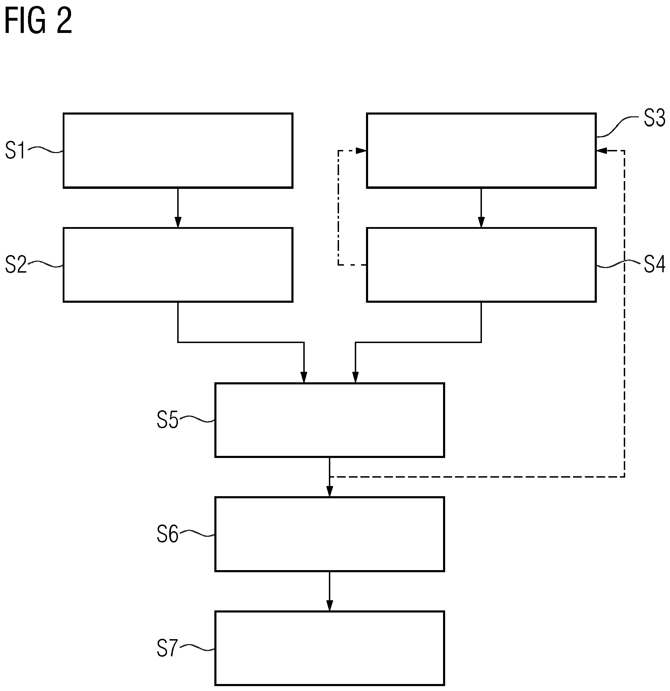

Fig. 2 zeigt eine weitere Variante eines Verfahrens zum Bestimmen eines bevorzugten Zielorts für ein Fahrzeug 100, insbesondere einen Krankenwagen, aufweisend ein medizinisches Bildgebungsgerät 101. In diesem Beispiel kann der Schritt des ersten Bestimmens S3 und des zweiten Bestimmens S4 vorzugsweise wiederholt stattfindet, so dass bei einer aktualisierten Position 4 des Fahrzeugs 100 eine aktualisierte Anzahl an optionalen Zielorten 1,2,3 bestimmt werden kann.

Weiterhin findet der Schritt des ersten Bestimmens S3 und der Schritt des zweiten Bestimmens S4 zumindest teilweise im Wesentlichen zeitgleich mit dem Schritt des Bewertens S2 statt, so dass vorzugsweise mit dem Abschluss des Schritts des Bewertens S2 oder zumindest in zeitlicher Nähe dazu bereits eine Anzahl an optionalen Zielorte 1,2,3 für das dritte Bestimmen S5 bereitgestellt werden kann. Außerdem kann der Schritt des ersten Bestimmens S3 und/oder der Schritt des zweiten Bestimmens S4 auch bereits im Wesentlichen zeitgleich oder während eines Schritts des Erzeugens des ersten Bilddatensatzes S0 stattfinden bzw. gestartet werden.

Es ist auch denkbar, wie hier durch den gestrichelten Pfeil angedeutet, dass selbst nach einem Schritt des dritten Bestimmens S5 wiederholt ein erstes Bestimmen S3 und zweites Bestimmen S4 stattfindet, so dass bei Änderungen zumindest eines Parameters, auf welchem das dritte Bestimmen S5 des bevorzugten Zielorts basiert, ein aktueller und gegebenenfalls optimierter bevorzugter Zielort ausgegeben werden kann. Ein solches Szenario kann beispielsweise ein durch einen Unfall unerwartet auftretender Stau sein, welcher potenziell zu einer Verzögerung führt, oder eine kurzfristige Änderung der Verfügbarkeit der medizinischen Ausstattung an dem Zielort.

Außerdem weist das hier gezeigte beispielhafte Verfahren außerdem den Schritt S7 des Ermittelns von Navigationsinformationen zur Erreichung des bevorzugten Zielorts, welche beispielsweis in visueller und/oder akustischer Form mittels einer Ausgabeeinheit 119 ausgegeben werden, so dass ein Fahrer des Fahrzeug 100 mittels der Navigationsinformation zu dem bevorzugten Zielort geleitet werden kann.

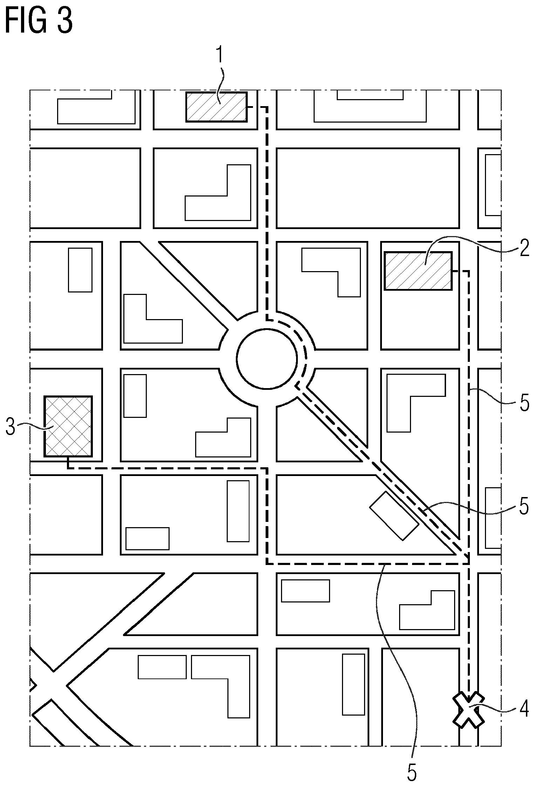

Fig. 3 zeigt zur Veranschaulichung schematisch eine Kartendarstellung, in welcher eine bestimmten Position 4 des Fahrzeugs 100 markiert ist. Des Weiteren sind exemplarisch drei optionale Zielorte 1,2,3 durch Schraffierung hervorgehoben. Die optionalen Zielorte 1,2,3 markieren jeweils optionale Zielorte 1,2,3 für die Behandlung des Patienten, welche zumindest basierend auf der Position 4 des Fahrzeugs bestimmt wurden.

Die unterschiedliche Schraffierung der optionalen Zielorte 1 und 2 gegenüber dem optionalen Zielort 3 soll in diesem Fall verdeutlichen, dass die optionalen Zielorte 1 und 2 beispielsweise eine andere medizinische Ausstattung, beispielsweise ein anderes Ausstattungslevel, aufweisen als der optionale Zielort 3. Beispielsweise stellen die optionalen Zielorte 1 und 2 CSCs/TCSCs und der optionale Zielort 3 ein PSC dar. In gleicher Weise kann die Schraffierung auch unterschiedliche Verfügbarkeiten der medizinischen Ausstattung oder auch anderweitige Parameter symbolisieren. Beispielsweise ist der optionale Zielort 3 für eine Behandlung eines Patienten momentan wegen der Auslastung der medizinischen Ausstattung am Behandlungsort nicht verfügbar.

Außerdem sind jeweils Fahrrouten 5 zu einem jeweiligen optionalen Zielort 1,2,3 der Anzahl an optionalen Zielorten 1,2,3. Basierend auf der Fahrtroute kann gegebenenfalls eine Fahrtstrecke oder auch eine vorhergesagte Fahrtdauer, welche das auch das aktuelle oder vorhergesagte Verkehrsaufkommen auf der Fahrtroute miteinbeziehen kann, bestimmt sein.

Je nach Bewertung des Bilddatensatzes kann dann aus der Anzahl an optionalen Zielorten 1,2,3 der bevorzugte Zielort ausgewählt werden. Ist der Patient ein Schlaganfallpatient und umfasst die Bewertung des Bilddatensatzes beispielsweise die Diagnose einer LVO, kann basierend auf der aktuellen Position 4 des Fahrzeugs 100 das schnellstmöglich erreichbare und für eine Behandlung verfügbare CSC/TCSC, im obig geschilderten Fall z.B. Zielort 2 oder gegebenenfalls auch Zielort 3, ausgewählt und als bevorzugter Zielort ausgegeben werden. Umfasst die Bewertung lediglich eine Diagnose, welche einen geringeres Ausstattungslevel notwendig macht, kann beispielsweise das PSC, im obig geschilderten Fall Zielort 3, als bevorzugter Zielort bestimmt werden.

Basierend auf dem bestimmten bevorzugten Zielort können dann Navigationsinformationen zur Erreichung des bevorzugten Zielorts ermittelt werden und mittels einer Ausgabeeinheit ausgegeben werden. Die Navigationsinformationen können mittels einer ähnlichen Darstellung wie hier in Fig. 3 schematisch gezeigt als Kartendaten für einen Fahrer bereitgestellt werden. Beispielsweise umfasst die Recheneinheit oder das Fahrzeug 100 ein Navigationssystem 115, wobei der bevorzugte Zielort automatisch mittels der Schnittstelle als ausgewähltes Navigationsziel in das Navigationssystem 115 programmiert wird, welches darauf basierend die Navigationsinformation ermittelt. Die Navigationsinformation kann dabei visuelle Information, beispielweise wie hier dargestellt eine Karte, welche den bevorzugten Zielort und/oder eine Route zu dem bevorzugten Zielort ausgehend von der aktuellen Position des Fahrzeugs anzeigt, mittels einer Ausgabeeinheit in Form eines Displays ausgeben. Der Fahrer kann dann anhand von dem Navigationssystem 115 bereitgestellten Navigationsinformationen zu dem bevorzugten Zielort geleitet werden.

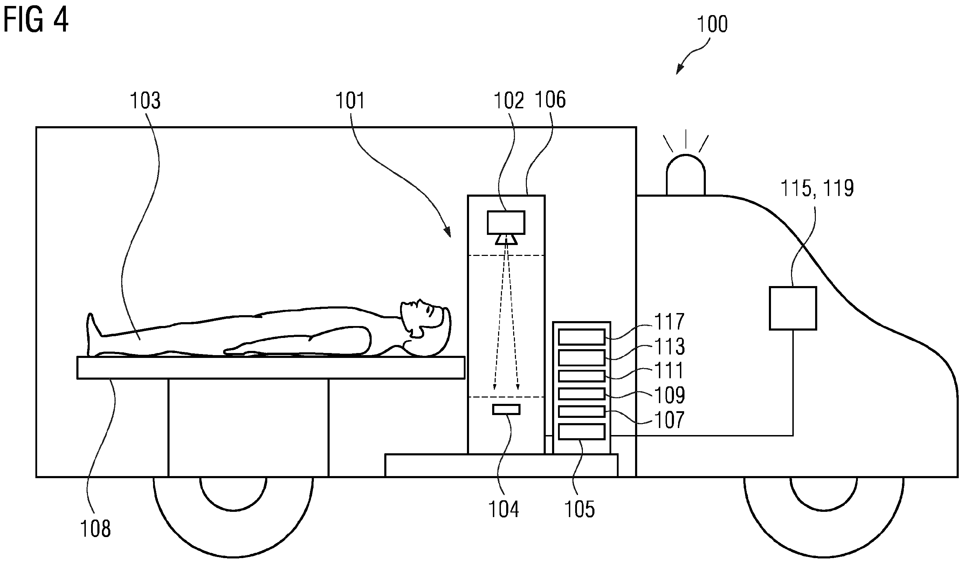

Fig. 4 zeigt ein Fahrzeug, insbesondere einen Krankenwagen, umfassend ein medizinisches Bildgebungsgerät 101. In diesem Beispiel ist das medizinische Bildgebungsgerät 101 ein Computertomographie-Gerät ist mit einer Messdatenaufnahmeeinheit 102,104 umfassend einen Röntgendetektor 104 und in Gegenüberstellung eine Röntgenquelle 102, welche in einer Gantry 106 angeordnet sind, welche eine Rotation der Messdatenaufnahmeeinheit 102,104 um eine gemeinsame Achse und damit die Aufnahme von Messdaten, d.h. insbesondere Röntgen-Projektionsmessdaten, eines Patienten 103 aus unterschiedlichen Winkelbereichen ermöglicht. Basierend auf diesen Messdaten kann dann ein erster oder zweiter dreidimensionaler Bilddatensatz, z.B. mittels eines Rekonstruktionsalgorithmus der gefilterten Rückprojektion, oder entsprechende Schichtbilddatensätze rekonstruiert werden.

Der Patient 103 ist dabei auf einer Patientenlagerungsvorrichtung 108 des medizinischen Bildgebungsgeräts 101 gelagert. Für die Aufnahme der Messdaten wird die Messdatenaufnahmeeinheit 102,104 relativ zum Patienten 103 positioniert, so dass ein Untersuchungsbereich 6,7 mittels der Messdatenaufnahmeeinheit 102,104 abgetastet werden kann. Eine Positionierung kann durch ein Verfahren oder Positionieren der Patientenlagerungsvorrichtung 108 und/oder auch durch ein Verfahren oder Positionieren der Messdatenaufnahmeeinheit 102,104, d.h. im Wesentlichen der Gantry 106, ermöglicht werden.

Das medizinische Bildgebungsgerät 101, hier in Form des CT-Geräts, ist insbesondere ausgebildet einen ersten Bilddatensatz des Patienten 103 zu erzeugen, indem es ausgebildet ist Messdaten aufzunehmen und basierend auf den Messdaten den Bilddatensatz zu rekonstruieren. Das medizinischen Bildgebungsgeräts 101 kann insbesondere dazu ausgebildet sein einen CT-Angiographie-Bilddatensatz zu erzeugen. Ein CT-Angiographie-Bilddatensatz erlaubt besonders vorteilhaft den Bilddatensatz auf das Vorhandensein eines Gefäßverschlusses zu prüfen und den Ort des Gefäßverschlusses zu lokalisieren und/oder die Art des vom Gefäßverschluss betroffenen Blutgefäß zu bestimmen. Ein Angiographie-Bilddatensatz erlaubt daher besonders vorteilhaft ein Bewerten des Bilddatensatzes im Rahmen einer Behandlung eines Schlaganfallpatienten. Es sind jedoch auch andere Bilddatensätze, beispielsweise native CT-Bilddatensätze ohne Kontrastmittelunterstützung oder Perfusions-Bilddatensätze, und ebenso auch andere Anwendungsfälle im Rahmen des Verfahrens denkbar.

Das Fahrzeug 100 weist außerdem eine erste Schnittstelle 105 auf, ausgebildet den erzeugten Bilddatensatz für eine Weiterverarbeitung mittels der Recheneinheit 107 bereitzustellen.

Das Fahrzeug 100 umfasst außerdem eine Recheneinheit 107, ausgebildet eine Position 4 des Fahrzeugs zu bestimmen. Die Recheneinheit ist außerdem ausgebildet eine Anzahl an optionalen Zielorten 1,2,3 für eine Behandlung des Patienten 103 zumindest basierend auf der bestimmten Position 4 des Fahrzeugs 100 zu bestimmen. Die Recheneinheit 107 ist außerdem ausgebildet zum dritten Bestimmen S5 des bevorzugten Zielorts aus der Anzahl an optionalen Zielorte 1,2,3 zumindest basierend auf dem bewerteten Bilddatensatz und der bestimmten Position 4 des Fahrzeugs 101.

Die Recheneinheit 107 kann in Form eines Computers, eines Mikrocontrollers oder eines integrierten Schaltkreises umgesetzt sein. Die Recheneinheit 107 kann Hardware-Elemente oder Software-Elemente aufweisen, beispielsweise einen Mikroprozessor oder ein sogenanntes FPGA (englisches Akronym für "Field Programmable Gate Array"). Die Recheneinheit 107 kann auch als Verbund an Computern umgesetzt sein. Das Fahrzeug 100 umfasst außerdem eine zweite Schnittstelle 109, ausgebildet den bestimmten, bevorzugten Zielort auszugeben.

Das Fahrzeug 100 weist in diesem Beispiel außerdem ein Navigationssystem 115 auf, welches ausgebildet ist Navigationsinformationen zu einem Navigationsziel zu ermitteln und mittels einer Ausgabeeinheit 119, beispielsweise in Form eines Displays oder eines Lautsprechers, auszugeben. Der von der Recheneinheit 107 bestimmte bevorzugte Zielort kann dann insbesondere mittels der zweiten Schnittstelle 109 an das Navigationssystem 115 ausgegeben werden. Das Navigationssystem 115 kann gegebenenfalls auch die Positionsbestimmungseinheit umfassen und für die Recheneinheit 107 abrufbar bereitstellen.

Bei der ersten Schnittstelle 105 und der zweiten Schnittstelle 109 kann es sich um eine Hardware- oder Softwareschnittstelle handeln (beispielsweise PCI-Bus, USB oder Firewire).

Die Vorrichtung kann außerdem zumindest eine Speichereinheit 117 umfassen. Es kann auch eine Speichereinheit von der Recheneinheit umfasst sein. Diese kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Die zumindest eine Speichereinheit 117 kann vorgesehen sein, den ersten Bilddatensatz für einen Schritt des Bewertens zwischenzuspeichern. Die zumindest eine Speichereinheit 117 kann auch vorgesehen sein, beispielsweise eine Datenbank an Behandlungsorten und gegebenenfalls mit diesen verknüpften Informationen für ein Abrufen mittels der Recheneinheit vorzuhalten. Die zumindest eine Speichereinheit 117 kann auch vorgesehen sein, die bestimmte Anzahl an optionalen Zielorten 1,2,3 für einen folgenden Schritt des dritten Bestimmens zwischenzuspeichern.

Das Fahrzeug 100 weist in diesem Beispiel außerdem eine Kommunikationseinheit 111 auf. Die Kommunikationseinheit 111 kann beispielsweise erlauben über ein Funknetz Daten, d.h. Informationen über die Behandlungsorte oder optionalen Zielorte 1,2,3 von einer zentralen Einheit abzufragen, beispielsweise hinsichtlich einer medizinischen Ausstattung oder einer Verfügbarkeit. Die Kommunikationseinheit kann zum Beispiel ein Telemedizinsystem sein, welche die Verbindung und Datenübertragung über zumindest ein Funknetz, insbesondere ein Mobilfunknetz, erlaubt.

Das Fahrzeug 100 weist in diesem Beispiel außerdem eine Positionsbestimmungseinheit 113 auf, beispielsweise in Form eines GPS Empfängers, welcher ausgebildet ist, die Position des Fahrzeugs zu bestimmen und für Weiterverarbeitung abrufbar für die Recheneinheit bereitzustellen.

Der Gegenstand der Erfindung ist nicht auf die vorstehend beschriebenen Ausführungsbeispiele beschränkt. Vielmehr können weitere Ausführungsformen von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

## Patentansprüche

1. Verfahren zum Bestimmen eines bevorzugten Zielorts für ein Fahrzeug (100), insbesondere einen Krankenwagen, aufweisend ein medizinisches Bildgebungsgerät (101), umfassend die Schritte,
- Bereitstellen (S1) eines Bilddatensatzes eines Patienten (103), welcher mittels des medizinischen Bildgebungsgeräts (101) erzeugt wurde, mittels einer ersten Schnittstelle (105),
- Bewerten (S2) des bereitgestellten Bilddatensatzes mittels einer Recheneinheit (107),
- Erstes Bestimmen (S3) einer Position (4) des Fahrzeugs (100) mittels der Recheneinheit (107),
- Zweites Bestimmen (S4) einer Anzahl an optionalen Zielorten (1,2,3) für eine Behandlung des Patienten (103) zumindest basierend auf der bestimmten Position (4) des Fahrzeugs (100) mittels der Recheneinheit (107),
- Drittes Bestimmen (S5) des bevorzugten Zielorts aus der Anzahl an optionalen Zielorten (1,2,3) zumindest basierend auf dem bewerteten Bilddatensatz und der bestimmten Position (4) des Fahrzeugs (100) mittels der Recheneinheit (107),
- Ausgeben (S6) des ausgewählten, bevorzugten Zielorts mittels einer zweiten Schnittstelle (109).

2. Verfahren nach Anspruch 1, wobei im Schritt des zweiten Bestimmens (S4) außerdem zumindest ein Parameter der folgenden Liste bestimmt wird
- Medizinische Ausstattung eines jeweiligen optionalen Zielorts (1,2,3) für die Behandlung des Patienten (103),
- Verfügbarkeit der medizinischen Ausstattung eines jeweiligen optionalen Zielorts (1,2,3) für die Behandlung des Patienten (103),
- Fahrroute (5) zu einem jeweiligen optionalen Zielort (1,2,3),
- Fahrtdauer zu einem jeweiligen optionalen Zielort (1,2,3).

3. Verfahren nach einem der Ansprüche 1 oder 2, wobei der Schritt des Bewertens (S2) des Bilddatensatzes umfasst, den Bilddatensatz auf das Vorliegen eines Gefäßverschlusses eines Blutgefäßes des Patienten (103) zu prüfen.

4. Verfahren nach Anspruch 3, wobei bei Vorliegen eines Gefäßverschlusses außerdem ein Ort des Gefäßverschlusses oder eine Art des vom Gefäßverschluss betroffenen Blutgefäß bestimmt wird.

5. Verfahren nach einem der vorstehenden Ansprüche, wobei der Schritt des Bewertens (S2) das Anwenden einer trainierten Funktion umfasst.

6. Verfahren nach einem der vorstehenden Ansprüche, wobei der erzeugte Bilddatensatz ein CT-Angiographie-Bilddatensatz ist.

7. Verfahren nach einem der vorstehenden Ansprüche, wobei der Schritt des ersten Bestimmens (S3) und der Schritt des zweiten Bestimmens (S4) wiederholt werden, so dass bei einer aktualisierten Position (4) des Fahrzeugs (100) eine aktualisierte Anzahl an optionalen Zielorten (1,2,3) bestimmt wird.

8. Verfahren nach einem der vorstehenden Ansprüche, wobei der Schritt des ersten Bestimmens (S3) und/oder der Schritt des zweiten Bestimmens (S4) während des Schritts des Bewertens (S2) durchgeführt wird.

9. Verfahren nach einem der vorstehenden Ansprüche, wobei basierend auf dem bestimmten bevorzugten Zielort Navigationsinformationen zur Erreichung des bevorzugten Zielorts ermittelt und mittels einer Ausgabeeinheit (119) ausgegeben werden.

10. Fahrzeug (100), insbesondere Krankenwagen, umfassend
- ein medizinisches Bildgebungsgerät (101), ausgebildet einen Bilddatensatz eines Patienten (103) zu erzeugen,
- eine erste Schnittstelle (105), ausgebildet den erzeugten Bilddatensatz für eine Weiterverarbeitung bereitzustellen,
- eine Recheneinheit (107), ausgebildet
• den erzeugten Bilddatensatz zu bewerten,
• eine Position (4) des Fahrzeugs (101) zu bestimmen,
• eine Anzahl an optionalen Zielorten für eine Behandlung des Patienten (103) zumindest basierend auf der bestimmten Position (4) des Fahrzeugs (100) zu bestimmen,
• einen bevorzugten Zielort (3) aus der Anzahl an optionalen Zielorten (1,2,3) zumindest basierend auf dem bewerteten Bilddatensatz und der bestimmten Position (4) des Fahrzeugs (100) auszuwählen,
- eine zweite Schnittstelle (109), ausgebildet den ausgewählten, bevorzugten Zielort auszugeben.

11. Fahrzeug (100) nach Anspruch 10, außerdem ausgebildet ein Verfahren nach einem der Ansprüche 2 bis 9 auszuführen.

12. Computerprogramm, welches direkt in einen Speicher einer Recheneinheit (107) eines Fahrzeugs (100), insbesondere eines Krankenwagens, ladbar ist, mit Programmabschnitten, um alle Schritte des Verfahrens nach einem der Ansprüchen 1 bis 9 auszuführen, wenn die Programmabschnitte von der Recheneinheit (107) ausgeführt werden.

13. Computerprogrammprodukt mit einem Computerprogramm, welches direkt in einen Speicher einer Recheneinheit (107) eines Fahrzeugs (100), insbesondere eines Krankenwagens, ladbar ist, mit Programmabschnitten, um alle Schritte des Verfahrens nach einem der Ansprüchen 1 bis 9 auszuführen, wenn die Programmabschnitte von der Recheneinheit (107) ausgeführt werden.

14. Computerlesbares Speichermedium, auf welchem von einer Recheneinheit (107) lesbare und ausführbare Programmabschnitte gespeichert sind, um alle Schritte des Verfahrens nach einem der Ansprüche 1 bis 9 auszuführen, wenn die Programmabschnitte von der Recheneinheit (107) ausgeführt werden.
